## Europäisches Patentamt
## European Patent Office
## Office européen des brevets

(19)

(11) Veröffentlichungsnummer : **0 034 762**
**B1**

(12)

# EUROPÄISCHE PATENTSCHRIFT

(45) Veröffentlichungstag der Patentschrift :
15.12.82

(21) Anmeldenummer : **81100972.9**

(22) Anmeldetag : **12.02.81**

(51) Int. Cl.$^3$ : **C 07 C121/22, C 07 C120/00**

(54) **Verfahren zur Gewinnung von Malonsäuredinitril.**

(30) Priorität : **21.02.80 DE 3006492**

(43) Veröffentlichungstag der Anmeldung :
**02.09.81 (Patentblatt 81/35)**

(45) Bekanntmachung des Hinweises auf die Patenterteilung : **15.12.82 Patentblatt 82/50**

(84) Benannte Vertragsstaaten :
**AT BE DE FR GB IT NL SE**

(56) Entgegenhaltungen :
**DE A 2 449 013**

(73) Patentinhaber : **Degussa Aktiengesellschaft**
**Weissfrauenstrasse 9**
**D-6000 Frankfurt am Main 1 (DE)**

(72) Erfinder : **Kleemann, Axel, Dr.**
**Greifenhagenstrasse 25**
**D-6450 Hanau 9 (DE)**
Erfinder : **Schalke, Peter M., Dr.**
**Südring 74**
**D-6458 Rodenbach (DE)**

EP 0 034 762 B1

## Verfahren zur Gewinnung von Malonsäuredinitril

Malonsäuredinitril wird bekanntlich durch Umsetzung von Chlorcyan und Acetonitril bei Temperaturen von 500 °C bis herauf zu 1 200 °C gewonnen, siehe z.B. US-PS 2 553 406, DE-OS 24 49 013, DE-PS 17 68 154, DE-PS 19 11 174 ; japanische bekanntgemachte Anmeldung 41 (1966)-16 506 ; US-PS 3 417 126.

Die Reaktion wurde meistens ohne Katalysator durchgeführt, doch ist schon Chlor als Katalysator verwendet worden, siehe die oben genannte japanische Anmeldung 41 (1966)-16 506 und US-PS 3 417 126. Auch Cyanwasserstoff — wenn auch in grösseren als katalytischen Mengen — wurde zur Erhöhung der Selektivität eingesetzt, siehe JAP-PS 49-4 207.

Der grosse Nachteil bei der Herstellung von Malonsäuredinitril liegt in der sehr schwierigen Isolierung des Malonsäuredinitrils aus dem Reaktionsgas.

Im allgemeinen wurde das Reaktionsgas so rasch wie möglich gequencht und darauf das Malonsäuredinitril durch fraktionierte Destillation gewonnen, s. z.B. US-PS 3 055 738. Das Quenchen wurde auch durch Verwendung von Hilfsflüssigkeiten wie Acetonitril selbst, das sowohl wasserfrei wie auch wasserhaltig sein konnte, wie auch durch Wasser oder das kondensierte Malonsäuredinitril selbst unterstützt, s. DE-PS 17 68 154.

Aus diesen Quenchlösungen konnte aber das Malonsäuredinitril nur mit Verlusten gewonnen werden.

Die Ursache für diese Verluste liegen in der geringen Stabilität des Malonodinitrils begründet, das bei Einwirkung starker Säuren oder Laugen sowie bei längerem Erhitzen über 100 °C explosionsartig zerfallen kann.

Wird Wasser oder werden wasserhaltige Kühlmittel eingesetzt, so wirkt die dadurch gebildete wässrige Salzsäurelösung durch starke Verseifung auf das gebildete Malonsäuredinitril ein. Aus diesem Grund wurde das Reaktionsgemisch während der Abkühlung gleichzeitig neutralisiert. Das dadurch bei dem Prozess entstehende salzhaltige Abwasser, das ausserdem hochtoxische Stoffe wie in Wasser gelöstes Acetonitril, nicht umgesetztes Chlorcyan etc. enthält, ist schwierig zu beseitigen.

Auch substituierte Malonsäuredinitrile, wie z.B. o-Chlorbenzalmalonsäuredinitril, wurden mit einer Kühlflüssigkeit hergestellt, in diesem Fall mit o-Chlorbenzaldehyd, in die das Reaktionsgas aus der Acetonitril- und Chlorcyan-Umsetzung eingeleitet wurde, s. US-PS 3 549 684. Ausbeute und Reinheit des Produktes werden aber nicht angegeben.

Da die Kühlmittel aus Gründen der besseren Verteilung in dem Reaktionsgas und damit zu dessen schnellerer Abkühlung in das Reaktionsgas eingespritzt wurden, kam noch hinzu, dass bei Verwendung der kondensierten Produktlösung selbst die Gefahr der Düsenverstopfung durch in dem kondensierten Produkt anwesende feste Verunreinigungen, wie z.B. Neutralisationsprodukte, bestand.

Obgleich die Fachwelt die Notwendigkeit einer besseren Ausbeute und einer leichteren Isolierung erkannt hatte, ging sie noch immer von dem Gedanken an eine Kondensation des Gesamtgases, möglichst durch Verwendung einer Kühlflüssigkeit, aus. Dieses Prinzip wurde auch noch einmal in jüngerer Zeit in der DE-PS 24 49 013 offenbart, in der allerdings die Kühltemperaturen auf mindestens 40 °C angehoben wurden. Als Kühlmittel diente die kondensierte Produktlösung selbst. Hierdurch soll die Harzbildung durch Verseifung des Malonsäuredinitrils auf Grund der anwesenden Salzsäure vermindert werden. Wenn auch die Ausbeuten, sowie die Betriebszeiten des Quenchteils und des ihm nachgeschalteten Destillationsteils verbessert wurden, so wurde die Harzbildung als solche nicht beseitigt.

Besonders nachteilig ist bei all diesen Verfahren, dass das thermisch empfindliche Malonsäuredinitril wegen seines höheren Siedepunktes bei der destillativen Aufarbeitung der Kondensationslösung erst nach dem Abdestillieren des im zwei- bis fünffachen Überschuss befindlichen Acetonitrils gewonnen werden kann. Es ist damit eine längere Zeit auf erhöhte Temperaturen erhitzt, wodurch auf Grund der thermischen Belastung Zersetzungsprodukte entstehen können und die Ausbeute absinkt.

Zweck der Anmeldung ist daher die Angabe eines technisch einfachen Verfahrens, das zur Erhöhung der Ausbeute des Malonsäuredinitrils aus dem Reaktionsgas führt.

Es wurde nun gefunden, dass man Malonsäuredinitril, das durch Umsetzung von Chlorcyan, gegebenenfalls von Cyanwasserstoff, hergestellt wurde, mit einer verbesserten Isolierungsmethode und in hoher Ausbeute aus dem Reaktionsgas gewinnen kann, wenn man das nach der Reaktion bei 500 bis 1 200 °C anfallende Reaktionsgas in mehreren aufeinanderfolgenden Temperaturstufen, bei denen die erste Temperaturstufe die höchste und die letzte Stufe die niedrigste Temperatur aufweisen, kondensiert und die jeweils anfallenden Kondensate getrennt aufarbeitet.

Sehr günstig lässt sich Malonsäuredinitril aus dem Reaktionsgas gewinnen, wenn man das Reaktionsgas derartig in den aufeinanderfolgenden Stufen kondensiert, dass man es — gegebenenfalls im Gleich- oder Gegenstrom mit Inertgas — in eine erste Kondensationsstufe, deren Temperaturgefälle 80 °C nicht unterschreiten und 170 °C nicht überschreiten soll, einführt, das Reaktionsgas in der ersten Kondensationsstufe fraktioniert kondensiert, das kondensierte rohe Malonsäuredinitril als Sumpfprodukt abzieht, den nicht kondensierten Anteil, der in der Hauptsache aus nicht umgesetztem Acetonitril, Chlorcyan sowie des während der Reaktion gebildeten Chlorwasserstoffes und niedrig siedenden Nebenprodukten besteht, in eine zweite Kondensationsstufe führt, bei einem Temperaturgefälle, das 20 °C nicht unterschreiten und 82 °C nicht überschreiten soll, bevorzugt zwischen 25 und 82 °C, kondensiert, das dabei anfallende zweite Kondensat aus restlichem Acetonitril und restlichem Chlorcyan abzieht und gegebenenfalls das hierbei anfallende Abgas aus Chlorwasserstoff, restlichen organischen Verunreinigungen, sowie gegebenenfalls Inertgas in eine dritte Kondensationsstufe einbringt, in der man den Chlorwasserstoff und gegebenenfalls das Inertgas bei Temperaturen von + 5 °C bis − 100 °C, bevorzugt von − 10 °C bis − 70 °C, von geringen Resten an organischen Verunreinigungen trennt, worauf man das aus der ersten Kondensationsstufe anfallende rohe Malonsäuredinitril in bekannter Weise destilliert und gegebenenfalls in bekannter Weise durch eine Umkristallisation aus niederen aliphatischen Alkoholen weiter reinigt ; das in der zweiten Kondensationsstufe anfallende Kondensat bevorzugt in die Reaktionsstufe zur Herstellung von Malonsäuredinitril zurückführt und gegebenenfalls den Chlorwasserstoff aus dem die dritte Kondensationsstufe verlassende Gas durch Auswaschen mit Wasser gewinnt.

Acetonitril und Chlorcyan werden — wie in den bekannten Verfahren — in Form ihrer Gase eingesetzt, ebenso in den bekannten Molverhältnissen, z.B. von 1 : 1 bis 6 : 1. Wenn Inertgase während der Fraktionierung verwendet werden sollen, so kommen hierfür besonders Stickstoff oder Kohlendioxid in Frage.

Für die erste und zweite Kondensationsstufe werden bekannte Reaktionskolonnen, die verschiedenartige Böden oder Füllungen enthalten, verwendet, bevorzugt Sambay- oder Fallfilmverdampfer. Bevorzugt wird das Reaktionsgemisch vorgekühlt in die 1. Stufe eingeleitet. Zur Einstellung der gewünschten Temperatur wird. z.B. eine thermostatisierte Flüssigkeit im Doppelmantel der Kolonnen oder Fallfilmverdampfer verwendet, die als unterste Temperatur in der ersten Kondensationsstufe 80 °C nicht unterschreiten und 170 °C nicht überschreiten soll.

Entsprechendes gilt für die zweite und dritte Kondensationsstufe, d.h., in der zweiten Stufe soll die Temperatur der Flüssigkeit 20 °C nicht unterschreiten und 82 °C nicht überschreiten ; in der dritten Stufe − 100 °C nicht unterschreiten und + 5 °C nicht überschreiten.

Als thermostatisierte Flüssigkeit kann in der ersten Kondensationsstufe auch Dampf für Heiztemperaturen über 100 °C verwendet werden. Im allgemeinen werden für dieses Temperaturgebiet die hierfür bekannten Öle eingesetzt.

Die optimalen Temperaturen für die Betreibung der Fraktionierkolonnen in den einzelnen Kondensationsstufen werden mit Hilfe analytischer Messungen durch einen Vorversuch festgelegt, da sie bekanntlich von üblichen Kolonnenparametern wie Höhe, Durchmesser, Art der Füllkörper oder Böden (wenn vorhanden), Einspeisemengen und Strömungsgeschwindigkeiten der zu kondensierenden Gase abhängen.

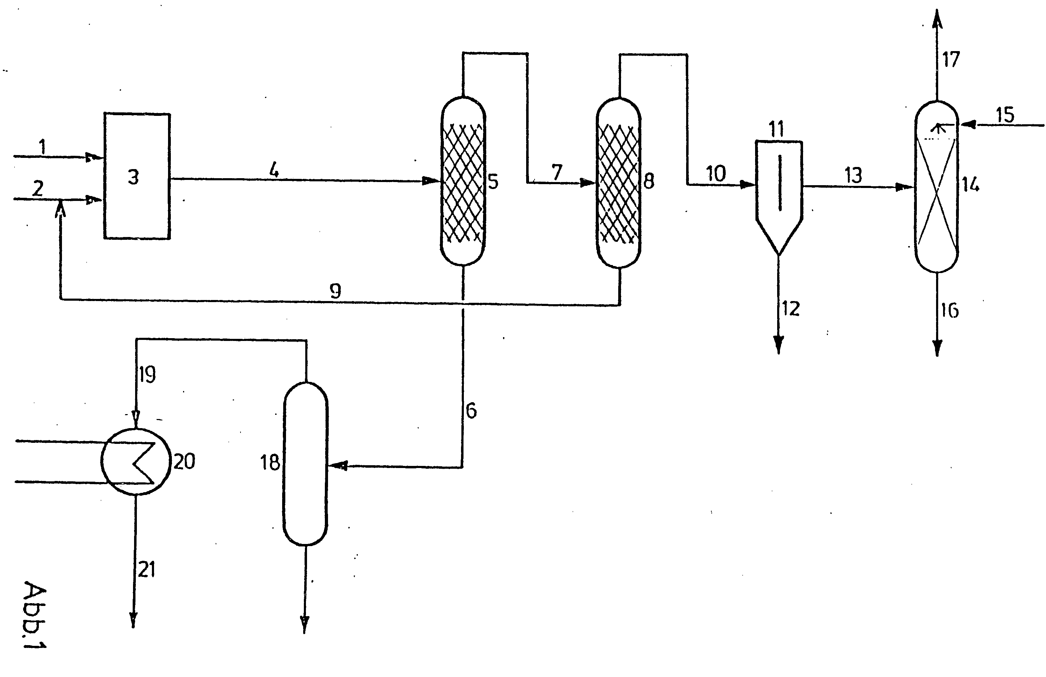

Die direkte Fraktionierung des Reaktionsgases wird anhand der schematischen Abbildung 1 erläutert.

Über Leitung 1 wird Chlorcyan, über Leitung 2 Acetonitril in den Reaktor 3 eingeführt und dort umgesetzt.

Aus dem Reaktor 3 tritt das malonsäuredinitrilhaltige Gas über die gekühlte Leitung 4 in die Fraktionierkolonne 5 ein, wo die Trennung in rohes Malonsäuredinitril, das über Leitung 6 abgenommen wird, und gasförmiges Acetonitril erfolgt, das zusammen mit ebenfalls gasförmigem Chlorwasserstoff, nicht umgesetztem Chlorcyan und organischen Nebenprodukten am Kopf der Kolonne 5 über Leitung 7 abgenommen und in die Fraktionierkolonne 8 geleitet wird.

Hier werden das nicht umgesetzte Acetonitril und Chlorcyan möglichst vollständig kondensiert und entweder als solche entnommen (nicht gezeigt) oder bevorzugt über Leitung 9 in den Reaktor 3 zur Herstellung von Malonsäuredinitril zurückgeführt.

Das die Kolonne 8 verlassende Restgas besteht im wesentlichen aus Chlorwasserstoff und gegebenenfalls Inertgas, sowie den organischen Nebenprodukten, wie Addukten aus Chlorcyan und Chlorwasserstoff mit unbekannter Struktur, sowie etwas Acetonitril.

In Abbildung 1 wird dieses Restgas über Leitung 10 in den Abscheider 11 geleitet und dort von den organischen Verunreinigungen befreit, die über Leitung 12 flüssig abgenommen und — falls gewünscht — gesondert aufgearbeitet werden können. (Letzteres nicht gezeigt).

Der jetzt von Verunreinigungen freie gasförmige Chlorwasserstoff gelangt über Leitung 13 in eine Waschkolonne 14, in die über Leitung 15 Wasser eingeführt wird. Die wässrige salzsaure Lösung verlässt über Leitung 16 das System. Je nach Menge des zugeführten Wassers können konzentrierte oder weniger konzentrierte salzsaure Lösungen gewonnen werden. Auch die Verwendung anderer Waschmittel wie alkalischer Lösungen, z.B. von verdünnten Alkalilaugen oder -Carbonatlösungen, ist an sich möglich ; sie führen aber nicht zu verwertbaren Produkten, wie bei der Verwendung von Wasser.

Die mögliche Verwendung von Inertgas, das über Leitung 17 das System verlässt, für die Aufarbeitung des Reaktionsgases wurde nicht gezeigt.

Das die Fraktionierkolonne 5 über Leitung 6 verlassende rohe Malonsäuredinitril tritt in die Fraktionierkolonne 18 ein, aus der das reine Malonsäuredinitril in Dampfform über Leitung 19 abgenommen und in dem Kühler 20 kondensiert wird. Das Produkt wird über Leitung 21 abgenommen.

Die dritte Kondensationsstufe 11 kann jedoch entfallen, wenn an einer Aufarbeitung des Restgases kein Interesse besteht, z.b. bei Vorliegen einer Vernichtungsanlage.

Für die erste und zweite Kondensationsstufe 5 und 8 werden bekannte Fraktionierkolonnen, die verschiedenartige Böden oder Füllungen enthalten, verwendet, bevorzugt Sambay- oder Fallfilmverdampfer. Sie werden unter Apparatedruck betrieben, bevorzugt Normaldruck. Die dritte Kondensationsstufe 11 besteht im allgemeinen aus einem Wärmetauscher, der mit Kühlsole betrieben wird. Diesem Wärmetauscher kann ein Aktivkohlefilter zur besseren Adsorption der Nebenprodukte vor- oder nachgeschaltet sein. Anstelle von Aktivkohle können auch andere Adsorptionsmittel verwendet werden.

Das Verfahren gestattet die direkte Isolierung des Malonsäuredinitrils aus dem Reaktionsgas unter Erhöhung der Ausbeute, ferner die direkte Isolierung des im Überschuss eingesetzten Acetonitrils, das dann sofort in einem Kreisprozess, gegebenenfalls mit darin enthaltenen Resten-an in der Reaktion nicht umgesetzten Chlorcyan in den Reaktor rezykliert werden kann, sowie die Gewinnung von salzsäurehaltigen Lösungen, die weiter einsatzfähig sind. Hierdurch fällt kein Abwasser an.

Beispiel 1

Über Leitung 1 werden pro Stunde 78,9 g (1,28 Mol) gasförmiges Chlorcyan und über Leitung 2 280,8 g (6,84 Mol) gasförmiges Acetonitril, sowie 1-2 l gasförmiger Stickstoff in den auf einem Meter mit einer Durchschnittstemperatur von 800 °C beheizten Reaktor 3 von einem Durchmesser von 55 mm eingespeist. Aus dem Reaktor 3 tritt das produkthaltige Gas über die gekühlte Leitung 4 in die Kolonne 5 ein. Die Kolonne 5 besteht aus einem Sambay-Verdampfer mit einer Innenoberfläche von 0,1 m² und einer aufgesetzten Füllkörperkolonne von 20 cm Länge. Der Doppelmantel der Kolonne ist mit heissem Öl durchflossen. Am Fuss der Kolonne werden pro Stunde 2-3 l gasförmiger Stickstoff eingespeist. Die Temperatur des Öls im Doppelmantel der Kolonne 5 ist so geregelt, dass am Fuss der Kolonne das in der Kolonne kondensierende Malonsäuredinitril-Rohprodukt mit einer Temperatur von etwa 160 °C über Leitung 6 entnommen werden kann, während am Kopf der Kolonne 5 das Abgas mit ca. 100 °C über Leitung 7 in die Fraktionierkolonne 8 geleitet wird. Die Kolonne 8 hat eine Länge von 50 cm, einen Durchmesser von 2 cm und ist mit Raschigringen gefüllt. Am Fuss der Kolonne 8 (nicht gezeigt) werden pro Stunde etwa 2-3 l gasförmiger Stickstoff eingeleitet. Die Aussenkühlung über Doppelmantel ist so geregelt, dass am Fuss der Kolonne 8 eine Sumpftemperatur bis zu 82 °C und am Kolonnenkopf eine Temperatur von 20-40 °C herrschen. Das in der Kolonne 8 kondensierte Acetonitril mit darin enthaltenen Resten an Chlorcyan wird am Fuss über Leitung 9 entnommen, sofort über Leitung 2 rückgeführt und vermischt mit frischem Acetonitril zur Ergänzung des in der Reaktion umgesetzten Acetonitrils in den Reaktor 3 geleitet.

Das die Kolonne 8 verlassende Restgas wird über Leitung 10 in einen bei − 30 °C gehaltenen Abscheider 11, bestehend aus einer Kühlfalle, geführt und dort von überschüssigem Acetonitril wie auch von den organischen Verunreinigungen befreit, die über Leitung 12 das System verlassen.

Das von den Verunreinigungen befreite Restgas gelangt über Leitung 13 durch ein Aktivkohlefilter (nicht gezeigt) in eine Waschkolonne 14, in der das Auswaschen des Restgases mit Wasser erfolgt. In der Kolonne 5 fallen pro Stunde 69,2 g Rohprodukt an, das zu 91,2 G% aus Malonsäuredinitril besteht und 4,8 G% Fumarsäure-/Maleinsäuredinitril und 0,7 G% Bernsteinsäuredinitril enthält. Acetonitril ist nicht nachweisbar. Das die Kolonne 5 verlassende Rohprodukt wird in der Fraktionierkolonne 18 aufdestilliert. Das bei 98-99 °C/13 mm siedende Malonsäuredinitril hat nach der Destillation eine Reinheit von 95-96 %.

In der Kolonne 8 fallen pro Stunde 223,9 g Lösung an, die zu 98,1 G% aus Acetonitril und zu 1,2 G% aus Chlorcyan besteht. Diese Lösung wird sofort in den Reaktor rückgeführt.

Im Abscheider 11 werden 13,2 g Lösung pro Stunde kondensiert. Diese Lösung hat einen Acetonitril-Gehalt von 56,2 % und muss separat aufgearbeitet werden.

Die Titration der wässrigen Lösung des Gaswäschers 14 mit Natronlauge gegen Methylorange als Indikator ergibt einen HCl-Gehalt, der 95,8 % der aus dem Chlorcyan entstandenen Salzsäure entspricht.

Aus diesen Ergebnissen errechnen sich Ausbeuten von 79,3 %, bezogen auf Acetonitril und 74,5 %, bezogen auf Chlorcyan.

Vergleichsbeispiel

Über Leitung 1 werden pro Stunde 78,9 g (1,28 Mol) gasförmiges Chlorcyan und über Leitung 2 280,8 g (6,84 Mol) gasförmiges Acetonitril, sowie 1-2 l gasförmiger Stickstoff in den auf einem Meter mit einer Durchschnittstemperatur von 800 °C beheizten Reaktor 3 von einem Durchmesser von 55 mm eingespeist.

Aus dem Reaktor 3 wird das produkthaltige Gas in eine Fraktionierkolonne geleitet, in der die Kondensation der gesamten Gasmenge bei 40 °C durchgeführt wird. Das diese Kolonne verlassende Restgas wird mit 40 °C warmer Produktlösung in einer Füllkörperkolonne ausgewaschen. Deren Abgas wird über einen bei − 30 °C gehaltenen Abscheider, bestehend aus einer Kühlfalle, geleitet, dann durch ein Aktivkohlefilter in eine mit Wasser gefüllte Waschkolonne geführt. Hier wird das Restgas HCl-frei gewaschen.

Durch Kondensation des Produktgases werden pro Stunde in Fraktionierkolonne und Abscheider 301,2 g Lösung erhalten, die durch Destillation aufgearbeitet werden. Bei einer Siedetemperatur von 82 °C werden aus einem einstündigen Versuch 229,3 g Acetonitril mit einer Reinheit von 98,9 % zurückerhalten. Anschliessend werden durch Vakuumdestillation bei 98-99 °C/13 Torr 61,8 g Malonsäuredinitril mit einer Reinheit von 92 % gewonnen. Das destillierte Produkt enthält weiterhin 6,3 % Fumarsäure/Malonsäuredinitril und 1,6 % Bernsteinsäuredinitril. Es errechnen sich Ausbeuten von 67,1 %, bezogen auf Chlorcyan und 68,6 %, bezogen auf Acetonitril.

Bei der Destillation wird aus der Lösung eine nicht unbeträchtliche Menge an Salzsäure sowie etwas Chlorcyan ausgetrieben. Die Titration des Inhalts der Waschkolonne mit Natronlauge gegen Methylorange als Indikator ergibt einen HCl-Gehalt, der nur 64,8 % der aus dem Chlorcyan entstandenen Salzsäure entspricht.

Im Vergleichsbeispiel wurde die Arbeitsweise nach DE-PS 24 49 013 nachgearbeitet.

Es ergibt sich, dass nach der erfindungsgemässen Arbeitsweise eine höhere Ausbeute und eine höhere Reinheit des isolierten Produktes erhalten werden. Dabei wird der Reaktor im Vergleichsbeispiel und im Beispiel nach dem erfindungsgemässen Verfahren in der gleichen Weise betrieben.

**Ansprüche**

1. Verfahren zur Gewinnung von Malonsäuredinitril, das durch Umsetzung von Acetonitril mit Chlorcyan, gegebenenfalls von Cyanwasserstoff, hergestellt wird und Aufarbeitung des Reaktionsgases, dadurch gekennzeichnet, dass man das nach der Umsetzung bei 500 bis 1 200 °C anfallende Reaktionsgas in mehreren aufeinanderfolgenden Temperaturstufen, bei denen die erste Temperaturstufe die höchste und die letzte Stufe die niedrigste Temperatur aufweisen, kondensiert und die jeweils anfallenden kondensate getrennt aufarbeitet.

2. Verfahren nach Anspruch 1, dadurch gekennzeichnet, dass man das Reaktionsgasgemisch derartig in den aufeinanderfolgenden Stufen kondensiert, dass man das Reaktionsgas — gegebenenfalls im Gleich- oder Gegenstrom mit Inertgas — in eine erste Kondensationsstufe, deren Temperaturgefälle 80 °C nicht unterschreiten und 170 °C nicht überschreiten soll, einführt, das Reaktionsgas in der ersten Kondensationsstufe fraktioniert kondensiert, das kondensierte rohe Malonsäuredinitril als Sumpfprodukt abzieht, den nicht kondensierten Anteil, der in der Hauptsache aus nicht umgesetztem Acetonitril, Chlorcyan sowie des während der Reaktion gebildeten Chlorwasserstoffes und niedrig siedender Nebenprodukten besteht, in eine zweite Kondensationsstufe führt, bei einem Temperaturgefälle, das 20 °C nicht unterschreiten und 82 °C nicht überschreiten soll, bevorzugt zwischen 25 und 82 °C, kondensiert, das dabei anfallende zweite Kondensat aus restlichem Acetonitril und restlichem Chlorcyan abzieht und gegebenenfalls das hierbei anfallende Abgas aus Chlorwasserstoff, restlichen organischen Verunreinigungen, sowie gegebenenfalls Inertgas in eine dritte Kondensationsstufe einbringt, in der man den Chlorwasserstoff und gegebenenfalls das Inertgas bei Temperaturen von + 5 °C bis

— 100 °C, bevorzugt von — 10 °C bis — 70 °C, von geringen Resten an organischen Verunreinigungen trennt, worauf man das aus der ersten Kondensationsstufe anfallende rohe Malonsäuredinitril in bekannter Weise destilliert und gegebenenfalls in bekannter Weise durch eine Umkristallisation aus niederen aliphatischen Alkoholen weiter reinigt ; das in der zweiten Kondensationsstufe anfallende Kondensat bevorzugt in die Reaktionsstufe zur Herstellung von Malonsäuredinitril zurückführt und gegebenenfalls den Chlorwasserstoff aus dem die dritte Kondensationsstufe verlassenden Gas durch Auswaschen mit Wasser gewinnt.

3. Verfahren nach Anspruch 1 und 2, dadurch gekennzeichnet, dass man das in der zweiten Kondensationsstufe anfallende Acetonitril, gegebenenfalls mit etwa darin enthaltenem restlichem Chlorcyan, direkt in den Reaktor zur Herstellung von Malonsäuredinitril zurückführt.

**Claims**

1. A process for the recovery of malonic acid dinitrile which is produced by the reaction of acetonitrile with cyanogen chloride or hydrogen cyanide, and working up the reaction gas, characterised in that the reaction gas resulting after the reaction at from 500 to 1 200 °C is condensed in several successive temperature stages, of which the first temperature stage has the highest temperature and the last stage has the lowest temperature, and the condensates resulting in each case are worked up separately.

2. A process according to claim 1, characterised in that the reaction gas mixture is condensed in the successive stages such that the reaction gas is introduced, optionally in co-current flow or in counterflow, with inert gas, into a first condensation stage, the drop in temperature of which should not fall below 80 °C and should not exceed 170 °C, the reaction gas is fractionally condensed in the first condensation stage, the condensed crude malonic acid dinitrile is drawn off as a bottom product, the uncondensed part which mainly consists of unreacted acetonitrile, cyanogen chloride and the hydrogen chloride formed during the reaction, and low-boiling byproducts, is introduced into a second condensation stage, is condensed at a drop in temperature which should not fall below 20 °C and should not exceed 82 °C, preferably being condensed at from 25 to 82 °C, the second condensate resulting thereform consisting of the remaining acetonitrile and remaining cyanogen chloride is drawn off, and optionally the waste gas resulting thereform of hydrogen chloride, remaining organic impurities and optionally inert gas is introduced into a third condensation stage, in which the hydrogen chloride and optionally the inert gas are separated from small residues of organic impurities at a temperature of from + 5 to — 100 °C, preferably from — 10 to — 70 °C, whereupon the crude malonic acid dinitrile resulting from the first condensation stage is distilled in a known manner and is optionally further purified in a known manner by recrystallisation from lower aliphatic alcohols ; the condensate resulting in the second condensation stage is preferably returned to the reaction stage for the production of malonic acid dinitrile and the hydrogen chloride is optionally obtained from the gas leaving the third condensation stage by washing with water.

3. A process according to claims 1 and 2, characterised in that the acetonitrile resulting in the second condensation stage, optionally with some remaining cyanogen chloride contained therein is returned directly into the reactor for the production of malonic acid dinitrile.

## Revendications

1. Procédé pour l'obtention du dinitrile de l'acide malonique, préparé par réaction de l'acétonitrile avec le chlorure de cyanogène, éventuellement de l'acide cyanhydrique, et traitement des gaz de la réaction, procédé caractérisé en ce que l'on condense les gaz réactionnels, obtenus après réaction à une température comprise entre 500 et 1 200 °C, à plusieurs degrés de température successifs, le premier degré constituant la température la plus élevée et le dernier, la température la plus basse, et que l'on traite chacun des gaz condensés séparément.

2. Procédé selon la revendication 1, caractérisé en ce que le mélange des gaz réactionnels est condensé dans les étapes successives, de telle façon que l'on introduit les gaz de réaction — éventuellement en commun ou à contre-courant avec un gaz inerte — dans une première étape de condensation, dont le gradient de température ne doit pas être inférieur à 80 °C ni supérieur à 170 °C, l'on procède à la condensation fractionnée des gaz de réaction dans la première étape de condensation, l'on soutire le dinitrile d'acide malonique brut condensé sous forme de produit de fond de colonne, l'on conduit la partie non condensée, qui est principalement constituée d'acétonitrile et de chlorure de cyanogène non réagis, ainsi que d'acide chlorhydrique formé au cours de la réaction et de produits secondaires à faible point d'ébullition, dans une deuxième étape de condensation, dont le gradient de température ne doit pas être inférieur à 20 °C ni supérieur à 82 °C, et de préférence comprise entre 25 et 82 °C, que l'on y condense ces gaz, que l'on soutire ce deuxième condensat formé d'acétonitrile résiduel et de chlorure de cyanogène résiduel et qu'éventuellement on conduit les gaz de rejet obtenus ici et composés d'acide chlorhydrique, d'impuretés organiques résiduelles, ainsi qu'éventuellement de gaz inerte, dans une troisième étape de condensation, où l'on sépare l'acide chlorhydrique et éventuellement le gaz inerte à des températures de + 5 °C à − 100 °C, de préférence de − 10 à − 70 °C, des faibles quantités résiduelles d'impuretés organiques, après quoi l'on distille, de manière connue, le dinitrile d'acide malonique brut obtenu dans la première étape de condensation, et on le purifie éventuellement de manière connue par recristallisation dans un alcool aliphatique à courte chaîne ; le produit condensé obtenu dans la deuxième étape de condensation étant recyclé dans la production de dinitrile malonique et éventuellement, l'acide chlorhydrique étant récupéré à partir des gaz sortant de la troisième étape de condensation par lavage à l'eau.

3. Procédé selon l'une des revendications 1 et 2, caractérisé en ce que l'acétonitrile obtenu dans la deuxième étape de condensation, avec éventuellement son contenu de cyanogène résiduel, est recyclé directement dans le réacteur de production du dinitrile malonique.

Abb.1